# EUROPEAN PATENT APPLICATION

(11) **EP 4 260 842 A1**
(43) Date of publication of application: **18.10.2023**
(21) Application number: 22168580.3
(22) Date of filing: 14.04.2022
(51) Int. Cl.: A61K 8/36, A61K 8/368, A61K 8/38, A61Q 11/02

(54) **DENTAL APPLIANCE CLEANSER**

(71) Applicant: GlaxoSmithKline Consumer Healthcare (UK) IP Limited, Brentford, Middlesex TW8 9GS (GB)
(72) Inventor: The designation of the inventor has not yet been filed
(74) Representative: Haleon Patent Department

(57) **Abstract**

The present invention relates to improved compositions for cleansing dental appliances comprising hydrogen peroxide and at least one acid selected from the group consisting of salicylic acid, furoic acid and formic acid.

## Description

### FIELD OF THE INVENTION

The present invention relates to improved compositions for cleansing dental appliances. The compositions are both aqueous and storage-stable, comprising at least one acid selected from the group consisting of salicylic acid, furoic acid and formic acid and low-levels of hydrogen peroxide. Particularly, the compositions are applied to dental appliances, outside of the wearer's mouth, in the form of an aerated foam.

### BACKGROUND TO THE INVENTION

Full or partial dentures are forms of dental appliance intended to be worn in the mouth as substitutes serving as a replacement for some or all of the teeth usually found in the oral cavity.

Like teeth, dentures and other dental appliances should be cleaned regularly for cosmetic reasons and to maintain good oral health. It is well known that microbiological deposition and proliferation on dentures and other dental appliances causes a number of problems for patients such as odour, denture-induced stomatitis, staining and unpleasant tastes within the oral cavity. However, unlike teeth, dentures and other dental appliances can be removed for cleaning. Generally, dental appliances are cleaned either by soaking them in a cleansing bath or by brushing with toothpaste or specially formulated cleansers.

Whilst soaking offers the advantage of reaching every part of the dental appliance, typically the cleansers used are sold in solid form, as a denture cleanser powder or tablet, or in concentrated liquid form. These solid forms need to be dissolved in water to form the cleansing bath following which a soaking period (e.g., overnight) is required to allow time for the cleanser solution to work. Such regimes are highly inconvenient and can leaves patients without their prostheses for extended periods. In contrast, mechanical brushing or scrubbing with creams and dentifrices is not always effective for removing bacteria and may scratch or damage the dental appliance surface.

Thus, there remains a need for further dental appliance cleansers that have an excellent safety and compatibility profile, but which are also convenient to use, fast-acting and compatible with denture materials.

### SUMMARY OF THE INVENTION

The Inventors have developed compositions with desirable cleansing properties, particularly for use with dental appliances such as dentures.

In a First Aspect, there is provided an aqueous dental appliance cleanser composition comprising (a) hydrogen peroxide, (b) at least one acid selected from the group consisting of salicylic acid, furoic acid and formic acid and (c) an anionic surfactant.

More particularly, aqueous compositions of the invention comprise (a) from about 0.05% to about 4% by weight of hydrogen peroxide, (b) from about 0.01% to about 0.25% by weight of at least one acid selected from the group consisting of salicylic acid, furoic acid and formic acid and (c) from about 0.05% to about 2.00% by weight of an anionic surfactant.

In some embodiments, compositions of the invention consist essentially of (a) from about 0.05% to about 4% by weight of hydrogen peroxide, (b) from about 0.01% to about 0.25% by weight of at least one acid selected from the group consisting of salicylic acid, furoic acid and formic acid and (c) from about 0.05% to about 2.00% by weight of an anionic surfactant.

The remainder or balance of the aqueous dental appliance cleanser composition is water. More particularly, the compositions comprise at least 80% by weight of water, for example at least 85% by weight or 90% by weight of water. More particularly, the compositions may comprise at least 91%, at least 92%, at least 93%, at least 94%, at least 95%, at least 96% or at least 97% by weight of water.

Exposure of a test suspension of either *Candida albicans* or *Klebsiella pneumoniae* (1-5 × 10⁸ cfu per ml) to a composition of the invention results in at least a log 2.5 reduction in the number of colony forming units within 60 seconds, preferably at least a log 3 reduction, more preferably at least a log 4 reduction, most preferably at least a log 5 reduction.

The compositions of the invention are storage-stable for a commercially reasonable period of time. More particularly, compositions of the invention are stable during storage and capable of the above-mentioned reductions in the number of colony forming units when measured 3 months after preparation. More preferably, compositions are capable of at least a log 2.5 reduction in the number of colony forming units within 60 seconds, preferably at least a log 3 reduction, more preferably at least a log 4 reduction, most preferably at least a log 5 reduction when measured 6 months after preparation, yet more preferably when measured 12 months after preparation and still yet more preferably when measured up to 24 months after preparation.

The compositions of the invention preferably have a pH within the range of from 2.8 to 3.5.

In some embodiments, compositions of the invention consist essentially of (a) from about 0.05% to about 4% by weight of hydrogen peroxide, (b) from about 0.01% to about 0.25% by weight of at least one acid selected from the group consisting of salicylic acid, furoic acid and formic acid and (c) from about 0.05% to about 2.00% by weight of an anionic surfactant, wherein the composition has a pH within the range of from 2.8 to 3.5.

Compositions of the invention may further comprise (d) a means for adjusting or maintaining the pH of the composition. In certain embodiments the compositions of the invention comprise a means for adjusting or maintaining the pH of the composition within the range of from 2.8 to 3.5.

In some embodiments, compositions of the invention consist essentially of (a) from about 0.05% to about 4% by weight of hydrogen peroxide, (b) from about 0.01% to about 0.25% by weight of at least one acid selected from the group consisting of salicylic acid, furoic acid and formic acid, (c) from about 0.05% to about 2.00% by weight of an anionic surfactant and (d) a means for adjusting or maintaining the pH of the composition, wherein the composition has a pH within the range of from 2.8 to 3.5.

The composition may comprise from about 0.01% to about 0.09% by weight of the means for adjusting or maintaining the pH of the composition. The means for adjusting or maintaining the pH of the composition may be an acid or a buffer. The composition may comprise from about 0.02% to about 0.08% by weight of the means for adjusting or maintaining the pH of the composition. Particularly, the means for adjusting or maintaining the pH of the composition is a buffer. More particularly, the means for adjusting or maintaining the pH of the composition is a buffer and the composition comprises from about 0.01% to about 0.09% by weight of a buffer. Yet more particularly, the means for adjusting or maintaining the pH of the composition is a buffer and the composition comprises from about 0.02% to about 0.08% by weight of a buffer. In some embodiments, the means for adjusting or maintaining the pH of the composition is a buffer comprising sodium citrate, more particularly, a sodium citrate buffer.

Thus, in another embodiment, there is provided an aqueous storage-stable dental appliance cleanser composition consisting essentially of: from about 0.05% to about 4% by weight of hydrogen peroxide, from about 0.01% to about 0.25% by weight of at least one acid selected from the group consisting of salicylic acid, furoic acid and formic acid, from about 0.05% to about 2.00% by weight of an anionic surfactant, from about 0.01% to about 0.08% by weight of a buffer particularly, a sodium citrate buffer, wherein the composition has a pH within the range of from 2.8 to 3.5.

Compositions of the invention may further comprise a sensory component. In certain embodiments, the sensory component is a flavouring agent and/or a colouring agent and/or an aroma agent.

In further embodiments, there is provided an aqueous storage-stable dental appliance cleanser composition consisting essentially of: from about 0.05% to about 4% by weight of hydrogen peroxide, from about 0.01% to about 0.25% by weight of at least one acid selected from the group consisting of salicylic acid, furoic acid and formic acid, from about 0.05% to about 2.00% by weight of an anionic surfactant, from about 0.01% to about 0.08% by weight of a buffer particularly, a sodium citrate buffer, wherein the composition has a pH within the range of from 2.8 to 3.5 and wherein the composition further comprises from about 0.01% to about 0.5% by weight of a sensory component.

In some embodiments the anionic surfactant is a sodium alkyl sulfate. In certain embodiments, the anionic surfactant is a sodium alkyl sulfate selected from the group consisting of sodium lauryl sulfate, sodium lauryl sulfonate and sodium laureth sulfate.

Thus, there is also provided an aqueous storage-stable dental appliance cleanser composition consisting essentially of: from about 0.05% to about 4% by weight of hydrogen peroxide, from about 0.01% to about 0.25% by weight of at least one acid selected from the group consisting of salicylic acid, furoic acid and formic acid, from about 0.05% to about 2.00% by weight of an anionic surfactant selected from the group consisting of sodium lauryl sulfate, sodium lauryl sulfonate and sodium laureth sulfate, from about 0.01% to about 0.08% by weight of a buffer particularly, a sodium citrate buffer, wherein the composition has a pH within the range of from 2.8 to 3.5 and wherein the composition further comprises from about 0.01% to about 0.5% by weight of a sensory component.

A further embodiment provides an aqueous storage-stable dental appliance cleanser composition consisting essentially of: from about 0.05% to about 4% by weight of hydrogen peroxide, from about 0.01% to about 0.25% by weight of at least one acid selected from the group consisting of salicylic acid, furoic acid and formic acid, from about 0.05% to about 2.00% by weight of an anionic surfactant, particularly an anionic surfactant selected from the group consisting of sodium lauryl sulfate, sodium lauryl sulfonate and sodium laureth sulfate, from about 0.01% to about 0.08% by weight of a buffer particularly, a sodium citrate buffer, wherein the composition has a pH within the range of from 2.8 to 3.5 and wherein the composition further comprises from about 0.01% to about 0.5% by weight of a sensory component.

In some embodiments, compositions of the invention comprise from about 0.1% to about 2% by weight of hydrogen peroxide, particularly from about 0.25% to 1% by weight of hydrogen peroxide.

Preferably, the at least one acid is one acid. Preferably the acid is salicylic acid. In some embodiments, compositions of the invention comprise from about 0.01% to 0.25% by weight of salicylic acid, particularly from about 0.1% to 0.2% by weight of salicylic acid.

In some embodiments, compositions of the invention comprise from about 0.25% to about 1% by weight of the anionic surfactant. In some embodiments, compositions of the invention comprise from about 0.25% to about 1.00% by weight of an anionic surfactant selected from the group consisting of sodium lauryl sulfate, sodium lauryl sulfonate and sodium laureth sulfate

Compositions of the invention are ready to use and do not require dilution or reconstitution. The compositions are foamable and may be applied to a surface in the form of an aerated foam. Compositions of the invention are substantially alcohol-free or alcohol-free. Compositions of the invention do not comprise ethylenediaminetetraacetate (EDTA).

In a Second Aspect of the invention, there is provided a container containing the composition of the First Aspect. Particularly, the container is a dispenser. Particularly, the dispenser comprises or consists essentially of, a container defining a reservoir and an applicator head in fluid communication with the reservoir, wherein the reservoir comprises the composition of the Invention and wherein the applicator head is configured to dispense the composition from the reservoir, particularly in the form of a spray and/or foam.

In certain embodiments, the dispenser comprises or consists essentially of, a container defining a reservoir and a foaming applicator head in fluid communication with the reservoir, wherein the reservoir comprises a composition according to the First Aspect and wherein the foaming applicator head is configured to dispense the composition from the reservoir in the form of a foam. By way of non-limiting example, the container comprises high density polyethylene (HDPE) and/or polyethylene terephthalate (PET). In some embodiments, the container comprises high density polyethylene (HDPE) and polyethylene terephthalate (PET) In some embodiments, the container is a high density polyethylene (HDPE) container. In some embodiments, the container is a polyethylene terephthalate (PET) container.

In a Third Aspect of the invention, there is provided a composition according to the First Aspect, for use in a method of cleansing dentures. There is also provided a method of cleansing a dental appliance outside the oral cavity comprising maintaining the dental appliance in contact with a composition according to the First Aspect for a period of time. There is also provided an aqueous dental appliance cleanser according to the First Aspect, for use in a method of killing a microorganism, such as *Candida albicans,* on the surface of a dental appliance. Particularly, the method comprises, contacting the microorganism on the surface of a dental appliance with the dental appliance cleanser for a period of time sufficient to kill the microorganism.

Particularly, the microorganism is a bacteria, fungi or virus. In some embodiments, the microorganism is a bacterium, particularly a gram-negative bacterium, such as *Klebsiella pneumoniae.* In some embodiments, the microorganism is a fungus, particularly a yeast, such as *Candida albicans.* In some embodiments, microorganism is a virus such as an enveloped or non-enveloped virus, for example an influenza virus or a coronavirus. In certain embodiments the virus is influenza virus H1N1. In certain embodiments the virus is a coronavirus such as SARS-CoV, SARS-CoV-1 or SARS-CoV-2.

### DESCRIPTION OF FIGURES

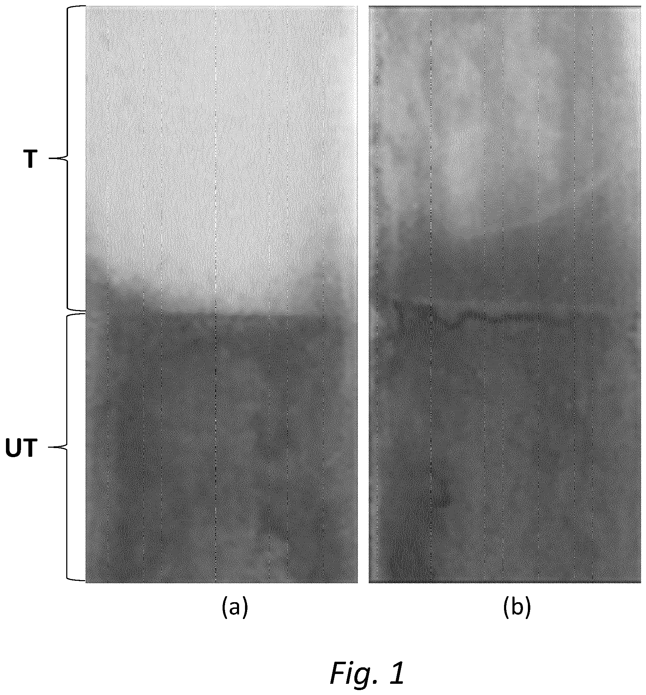

**FIGURE 1****:** Shows two ceramic tiles stained with tea comparing untreated sections (UT) with sections treated (T) with either (a) a composition of the invention or (b) commercial toothpaste.

### DETAILED DESCRIPTION OF THE INVENTION

The present invention is directed towards dental appliance cleansing compositions comprising at least one acid selected from the group consisting of salicylic acid, furoic acid or formic acid in combination with hydrogen peroxide and an anionic surfactant.

Surprisingly, the Inventors have discovered that such compositions are not only fast-acting but exhibit superior anti-microbial efficacy. Without wishing to be bound by theory, there appears to be a synergistic anti-microbial effect when micro-organisms are exposed to a combination of hydrogen peroxide and the acid component (selected from salicylic, formic and furoic acid) achieving (in a killing assay) log 2.5 or even greater reductions in the number of colony forming units within 60 seconds, even at low levels of hydrogen peroxide. It should be noted that other acids, such as phosphoric or citric acid, do not exhibit such synergistic anti-microbial efficacy and the effect is not linked to pKa, for example.

Advantageously, such compositions have improved dermal tolerance (compared with existing formulations on the market) making them particularly suitable for use in cleansing dental prostheses that are cleaned outside of the mouth where product contact with the skin of the hands and fingers is likely. The compositions are also effective at removing stains and highly surface compatible such that they do not corrode or tarnish plastic, such as acrylic resin, nylon, porcelain, resin, metal or other materials often found in full or partial dental prostheses and other dental appliances. Further, the formulations have low environmental impact, breaking down primarily into oxygen and water.

As used herein, the term "dental appliance" refers to dentures or partial dentures, artificial teeth, removable orthodontic bridges and denture plates, both upper and lower types, orthodontic retainers and appliances, protective mouthguards, nightguards to prevent bruxism and/or Temporomandibular joint (TMJ) disorder, and the like. The term "dental appliance cleanser" refers to a composition for use outside of the mouth to clean dental appliances, cleaning the surface and within the pores of a dental appliance, for example, killing bacteria and removing food particles, stains and other oral debris.

For the avoidance of doubt, reference to "% by weight" means by weight of the total composition, the amount of each ingredient being selected so that total ingredients in the composition sum to 100 percent by weight. Whilst not always explicitly listed as an ingredient, it should be understood that for aqueous solutions of the Invention, the remainder or balance of the composition is water.

The term, "aqueous solution" refers to a solution wherein the solvent is water and other constituents of the composition are dispersed, mixed, and/or dissolved therein. Particularly, the compositions comprise at least 80% by weight of water, for example at least 85% by weight or 90% by weight of water. More particularly, the compositions may comprise at least 91%, at least 92%, at least 93%, at least 94%, at least 95%, at least 96%, at least 97%, at least 98% or at least 99% by weight of water. Thus, the compositions may comprise from 80% to 99% by weight of water, such as 85% to 99%, 90% to 99% or 95% to 99% by weight of water. Particularly water is the only solvent and no additional organic solvents, such as alcohols, are used.

Compositions of the invention comprise hydrogen peroxide, also referred to as H₂O₂, having the CAS Number: 7722-84-1. Particularly, compositions of the invention comprise from about 0.05% to about 4% by weight of hydrogen peroxide, such as from about 0.05% to about 4%, from about 0.1% to about 4% or from about 0.25% to about 4% by weight of hydrogen peroxide. More particularly, from about 0.05% to about 2%, from about 0.1% to about 2% or about 0.25% to about 2% by weight of hydrogen peroxide. Yet more particularly, from about 0.05% to about 1%, from about 0.1% to about 1% or from about 0.25% to about 1% by weight of hydrogen peroxide. Still yet more particularly, from about 0.1% to about 0.75% or from about 0.25% to about 0.75%, such as about 0.25%, about 0.3%, about 0.4%, about 0.5%, about 0.6%, about 0.7% or about 0.75% by weight of hydrogen peroxide.

Compositions of the invention comprise at least one acid selected from the group consisting of salicylic acid, furoic acid and formic acid. Salicylic acid (CAS Number: 69-72-7) may also referred to as 2-Hydroxybenzoic acid. Formic acid (CAS Number: 64-18-6) may also be referred to as methanoic acid. Furoic acid refers to either 2-Furoic acid (CAS Number: 88-14-2), also called Furan-2-carboxylic acid, and 3-Furoic acid (CAS Number: 488-93-7), also called Furan-3-carboxylic acid. The composition may comprise 2-Furoic acid and/or 3-Furoic acid. In some embodiments, the acid is 2-Furoic acid. In other embodiments, the acid is 3-Furoic acid.

Whilst it is preferred to use one acid selected from the group consisting of salicylic acid, furoic acid and formic acid, a person skilled in the art will appreciate that combinations of these acids might also be used. For example, compositions of the invention further comprise at least two acids selected from the group consisting of salicylic acid, furoic acid and formic acid, for example two of these acids or three of these acids. Thus, in certain embodiments the compositions may comprise a combination of salicylic acid, furoic acid and formic acid, for example in any suitable molar ratio such as from 1:1:1 to 2:1:1, 1:2:1 or 1:1:2. In other embodiments, the compositions may comprise a combination of salicylic acid and furoic acid, a combination of salicylic acid and formic acid or a combination of furoic acid and formic acid, for example in any suitable molar ratio such as from 1:1 to 3:1, 1:1 to 1:1, such as 1:1, 1:2, 1:3, 3:1 or 2:1.

Particularly, compositions of the invention comprise an acid selected from the group consisting of salicylic acid, furoic acid and formic acid. In preferred embodiments of the invention, the acid is salicylic acid.

Compositions of the invention may comprise from about 0.01% to about 0.25% by weight of the at least one acid, from about 0.1% to about 0.25% by weight of the at least one acid or from about 0.15% to about 0.2% by weight of the at least one acid, for example about 0.15%, 0.16%, 0.17%, 0.18%, 0.19% or about 0.2% by weight of the at least one acid.

Compositions of the invention comprise a surfactant, more particularly an anionic surfactant. Suitable anionic surfactants are known in the art. Particularly, the anionic surfactant is a sodium alkyl sulfate, for example, a sodium sulfate having an alkyl group having 8 to 20 carbon atoms. Non-limiting examples of these include sodium decyl sulfate, sodium lauryl sulfate (also referred to as SLS or sodium dodecyl sulfate (SDS)), sodium tetradecyl sulfate, sodium hexadecyl sulfate and sodium sodium octadecyl sulfate. In certain embodiments, the anionic surfactant is a sodium alkyl sulfate selected from the group consisting of sodium lauryl sulfate, sodium lauryl sulfonate and sodium laureth sulfate. Particularly, the anionic surfactant is sodium lauryl sulfate.

The composition may comprise from about 0.05% to about 2.00% by weight of the anionic surfactant, particularly from about 0.1% to about 2% by weight, more particularly from about 0.25% to about 1% by weight or yet more particularly from about 0.3% to about 0.8% by weight of the anionic surfactant. For example, about 0.3%, 0.4%, 0.5%, 0.6%, 0.7% or about 0.8% by weight of the anionic surfactant.

Compositions of the invention preferably have a pH within the range of from 2.8 to 3.5, such as a pH within the range of from 2.8 to 3.2, for example a pH of about 3.0 or about 3.1. In order to achieve this, compositions of the invention may comprise a means for adjusting or maintaining the pH of the composition within this range. The composition may comprise from about 0.01% to about 0.09% by weight such as from about 0.02% to 0.08% by weight of the means for adjusting or maintaining the pH of the composition. The composition may comprise about 0.02%, 0.03%, 0.04%, 0.05%, 0.06%, 0.07% or about 0.08% by weight of the means for adjusting or maintaining the pH of the composition.

The means for adjusting or maintaining the pH of the composition may be an acid or a pH buffer (also referred to as a buffer). Particularly, the means for adjusting or maintaining the pH of the composition is a pH buffer. More particularly, the means for adjusting or maintaining the pH of the composition is a pH buffer comprising sodium citrate. Yet more particularly, the means for adjusting or maintaining the pH of the composition is a sodium citrate buffer. Sodium citrate buffers are known in the art and can be made and adjusted to the desired pH by mixing citric acid (monohydrate) and trisodium citrate (dehydrate), such as by way of non-limiting example, at ratios of from 82:18 to 73:27 respectively.

Particularly, compositions of the invention exhibit a dermal tolerance score of at least 10% mean cell viability, at least 11%, at least 12%, at least 13%, at least 14% or at least 15% mean cell viability as measured by in vitro dermal tolerance assay, more particularly as measured by the in vitro EPIDERM skin irritation test (EPI-200-SIT, MatTek Corporation). Particularly, compositions of the invention exhibit an improved dermal tolerance score (for example, a mean cell viability of at least 10% or higher) when compared with FRESHDENT denture foam (EverSmile).

Preferably, compositions of the invention are alcohol-free. The term "alcohol-free" refers to an absence of alcohol (0% by weight). For the avoidance of doubt, this term may include the presence of alcohol but in a quantity below the limit of detection of the analytical method and in an amount too low to be detected or accurately measured.

Alternatively, since some sensory components (discussed below) comprise alcohols, where a sensory component is included, compositions of the invention may be substantially alcohol-free. As used herein, "substantially alcohol free" means that the compositions contain less than 0.5% alcohol such as 0.4% or less, 0.3% or less, 0.25% or less, 0.2% or less, 0.1% or less, 0.05% or less or less than 0.01% alcohol.

Compositions of the invention are foamable formulations. A "foamable formulation" refers to a composition which is capable of forming a foam, particularly a foam having a small bubble size to give the appearance of a dense, rich foam The foam is capable of being brushed easily across the entire appliance.

The compositions of the invention form a stable foam having desirable physicochemical properties such as volume, density, and spread that do not change significantly over a period of time such that the foam does not break down immediately during use. More particularly, foams formed by compositions of the invention are not "quick-breaking" and will be stable when dispensed from a container and not breakdown immediately in contact with a surface, such as the surface of a dental appliance. Whilst foams formed by compositions of the invention will not be quick-breaking, they will be "breakable", in other words, able to be spread easily across the surface with application of mild shear force. Characteristics of foams can be measured using tests known in the art, such as by conducting foam formation (foam height vs time) and foam collapsibility experiments. Preferably the foam retains its structure such that it remains in contact with the surface for a time sufficient to cleanse and/or kill micro-organisms. Particularly, the duration of use is 3 minutes or less, more particularly 2 minutes or less, yet more particularly 60 seconds or less, for example between about 30 and 60 seconds.

Compositions of the current invention are foamable in the absence of a propellant. Particularly compositions of the invention are dispensed from a container without use of a propellant. Particularly, the compositions are foamable by manual aeration, for example where atmospheric air, is passed through the formulation to produce a foam by manual actuation of a dispensing head. Particularly, compositions of the invention may be dispensed from a container which has a suitable dispensing head, such as a foaming applicator head. The foam may be applied directly to the surface of a dental appliance, optionally with brushing. After cleansing, the dental appliance may be rinsed with water to remove the formulation before it is placed into the wearer's mouth.

For dental appliance cleansing compositions that are used to clean an appliance before it is placed in the mouth, it may be desirable to include at least one sensory component. A "sensory component" refers to a component that may be used to impart a flavour, fragrance, colour and/or sensation (such as cooling, warming or tingling) to the composition. Other than imparting the compositions with organoleptic properties that are desirable for consumers, generally the sensory component is not an active component, in other words it does not play an active role in cleansing or bacterial killing. Particularly, the sensory component is at least one flavour, fragrance and/or colouring agent. Sensory components are known in the art and may be natural or artificial (synthetic).

Compositions of the invention may comprise from about 0.01% to about 0.5% by weight of a sensory component, particularly from about 0.1% to about 0.3% by weight of a sensory component, more particularly from about 0.2% to about 0.3% by weight of a sensory component, such as about 0.1%, 0.15%, 0.2%, 0.25%, 0.3%, 0.35%, 0.4%, 0.45% or about 0.5% by weight of a sensory component.

The sensory component must be compatible with the constituents of the composition and not change or degrade, for example, in contact with hydrogen peroxide or acid. Thus, in certain embodiments compositions of the invention comprise at least one flavour component selected from the group consisting of (+)-menthol, (-)-menthol, (+)-isomenthol, (+)-neomenthol, (-)-neomenthol and (-)-neoisomenthol. Suitable sensory components are commercially available, for example, marketed under the name OPTAMINT, such as OPTAMINT 999529 GlacierFresh, by Symrise AG. In certain embodiments, compositions of the invention do not comprise (-)-isomenthol or (+)-neoisomenthol.

Typically, compositions of the invention are stable for at least 3 months at a room temperature of 20°C, particularly for at least 6 months at a room temperature of 20°C, more particularly for at least 12 months at a room temperature of 20°C and yet more particularly for at least 24 months at a room temperature of 20°C.

As used herein, the term "stable" refers to the ability of the composition to maintain certain physical or chemical properties, such as antimicrobial efficacy, at a specific storage temperature for a period of time after production.

The stability of compositions of the present invention may be assessed using antimicrobial efficacy as a proxy, any decrease over time indicative of degradation, for example. Thus stability may be assesses by comparing antimicrobial efficacy of a composition before and after storage using a suitable killing assay which measures the reduction in the number of live microorganisms present in a sample following exposure to the composition.

As used herein, the term "microorganism" refers to bacteria, fungi, such as yeasts or moulds, and viruses. Compositions of the Invention that kill and/or inhibit growth of bacteria, fungi and viruses and may be referred to as "antimicrobial" or "microbiocidal". Thus, compositions of the Invention may be "biocidal" compositions, killing and/or inhibiting growth of bacteria and/or "fungicidal" compositions, killing and/or inhibiting growth of fungi and/or "virucidal" compositions, inactivating virus particles such that the virus particles are unable to infect host cells.

As used herein, the term "kill" refers to causing death of a microorganism. In relation to a population of microorganisms, the term "kill" or "killing" refers to reducing the total amount or level of microorganisms by causing the death of some or all of the microorganisms in the population. With regard to viruses, kill or killing refers to rendering the virus incapable of infecting a host cell and/or incapable of replication in a host cell, for example by inactivation or destruction.

For the avoidance of doubt, use of the term "a" or "an" in relation to microorganisms such as bacteria, fungi and viruses is to be read to refer to one or more of that entity, for example a population of bacteria growing on a surface, such as in the form of a biofilm.

The killing assay may be a bacterial killing assay, a fungal killing assay or a viral killing or viral inactivation assay.

In some embodiments, the composition is a virucidal composition capable of killing an influenza virus or a coronavirus. In certain embodiments the virus is influenza virus H1N1. In certain embodiments the virus is a coronavirus such as SARS-CoV, SARS-CoV-1 or SARS-CoV-2.

Oropharyngeal candidosis (OPC) may be caused by opportunistic yeast species such as *Candida albicans.* It is often associated with wear of oral prosthetic appliances, such as dentures leading to denture-induced stomatitis, an inflammation of the oral mucosa. *C. albicans* is implicated as the main causative organism of denture-induced stomatitis. Therefore, the stability of compositions of the invention may be determined by measuring and comparing antimicrobial efficacy of a composition against *Candida albicans,* before and after storage, using a suitable killing assay such as the assay described in the Examples.

The level of killing may also be defined as a proportion, such as a percentage, or by reference to a log reduction in the total amount or level of microorganisms. Log reduction is used in the art to refer to the number of live microorganisms killed, for example following contact of the microorganisms with the aqueous dental appliance composition of the Invention. The terms "log X reduction" and "x log reduction" are used interchangeably to refer to a reduction in the number of live microorganisms of 10^{x}. For example, a log 4 reduction means that after contacting a population of microorganisms with the aqueous dental appliance cleanser, the number of remaining microorganisms in the population is 10,000 times less, i.e. 1/10⁴ or less of the population prior to contact with the aqueous dental appliance cleanser.

Particularly, exposure of a suspension of *Candida albicans*(10⁸ cfu per ml) to a composition of the invention yields at least a log 2.5 reduction, particularly at least a log 3 reduction, more particularly at least a log 4 reduction, yet more particularly at least a log 5 reduction, in the number of *Candida albicanscolony* forming units within 60 seconds of exposure when measured at least 3 months after preparation of the composition. Particularly, exposure of a suspension of *Candida albicans* (10⁸ cfu per ml) to a composition of the invention yields at least a log 2.5 reduction, particularly at least a log 3 reduction, more particularly at least a log 4 reduction, yet more particularly at least a log 5 reduction, in the number of *Candida albicans* colony forming units within 60 seconds of exposure when measured at least 6 months after preparation of the composition. Particularly, exposure of a suspension of *Candida albicans* (10⁸ cfu per ml) to a composition of the invention yields at least a log 2.5 reduction, particularly at least a log 3 reduction, more particularly at least a log 4 reduction, yet more particularly at least a log 5 reduction, in the number of *Candida albicans* colony forming units within 60 seconds of exposure when measured at least 12 months after preparation of the composition. Particularly, exposure of a suspension of *Candida albicans* (10⁸ cfu per ml) to a composition of the invention yields at least a log 2.5 reduction, particularly at least a log 3 reduction, more particularly at least a log 4 reduction, yet more particularly at least a log 5 reduction, in the number of *Candida albicans* colony forming units within 60 seconds of exposure when measured at least 24 months after preparation of the composition.

*Klebsiella pneumoniae* is a gram-negative bacteria that causes multiple healthcare-associated infections. Therefore, stability of compositions of the invention may also be determined by measuring antimicrobial efficacy against *Klebsiella pneumoniae* using a suitable bacterial killing assay such as the assay described in the Examples.

Particularly, exposure of a suspension of *Klebsiella pneumoniae* (10⁸ cfu per ml) to a composition of the invention yields at least a log 2.5 reduction, particularly at least a log 3 reduction, more particularly at least a log 4 reduction, yet more particularly at least a log 5 reduction, in the number of *Klebsiella pneumoniae* colony forming units within 60 seconds of exposure when measured at least 3 months after preparation of the composition. Particularly, exposure of a suspension of *Klebsiella pneumoniae* (10⁸ cfu per ml) to a composition of the invention yields at least a log 2.5 reduction, particularly at least a log 3 reduction, more particularly at least a log 4 reduction, yet more particularly at least a log 5 reduction, in the number of *Klebsiella pneumoniae* colony forming units within 60 seconds of exposure when measured at least 6 months after preparation of the composition. Particularly, exposure of a suspension of *Klebsiella pneumoniae* (10⁸ cfu per ml) to a composition of the invention yields at least a log 2.5 reduction, particularly at least a log 3 reduction, more particularly at least a log 4 reduction, yet more particularly at least a log 5 reduction, in the number of *Klebsiella pneumoniae* colony forming units within 60 seconds of exposure when measured at least 12 months after preparation of the composition. Particularly, exposure of a suspension of *Klebsiella pneumoniae* (10⁸ cfu per ml) to a composition of the invention yields at least a log 2.5 reduction, particularly at least a log 3 reduction, more particularly at least a log 4 reduction, yet more particularly at least a log 5 reduction, in the number of *Klebsiella pneumoniae* colony forming units within 60 seconds of exposure when measured at least 24 months after preparation of the composition.

Compositions of the invention retain at least 80%, at least 85%, at least 90%, at least 91%, at least 92%, at least 93%, at least 94%, at least 95%, at least 96%, at least 97%, at least 98% or at least 99% of their active ingredient content after at least three months, after at least six months, at least twelve months or at least twenty-four months at room temperature (20° C.).

There is also provided a container comprising an aqueous dental appliance cleanser composition of the invention. There is also provided a dispenser comprising (i) a container comprising an aqueous dental appliance cleanser composition of the invention and (ii) a foaming applicator head. More particularly, there is provided a dispenser consisting essentially of (i) a container comprising an aqueous dental appliance cleanser composition of the invention and (ii) a foaming applicator head configured to dispense the aqueous dental appliance cleanser composition in the form of a foam. Yet more particularly, there is provided a dispenser comprising or consisting essentially of, a container defining a reservoir and a foaming applicator head in fluid communication with the reservoir, wherein the reservoir comprises a cleanser composition of the invention and wherein the foaming applicator head is configured to dispense the aqueous dental appliance cleanser composition from the reservoir in the form of a foam.

The container may be manufactured from any suitable material. Suitable materials are those materials that do not degrade, interact, or react with the components of the dental appliance cleanser composition. Particularly, the suitable material does not interact, react with or degrade on contact with hydrogen peroxide, more particularly on contact, such as prolonged contact, with 0.05% to about 4% by weight of hydrogen peroxide. Particularly, the suitable material does not interact, react with or degrade on contact, such as prolonged contact, with salicylic acid, furoic acid or formic acid, more particularly from 0.01% to about 0.25% by weight of salicylic acid, fuoric acid or formic acid. Particularly, the suitable material does not interact, react with or degrade on contact, such as prolonged contact, with an anionic surfactant, more particularly from 0.05% to about 2.00% by weight of the anionic surfactant.

By way of non-limiting example, the container may comprise high density polyethylene (HDPE) and/or polyethylene terephthalate (PET). In some embodiments, the container comprises high density polyethylene (HDPE) and polyethylene terephthalate (PET). In some embodiments, the container is a high-density polyethylene (HDPE) container. In some embodiments, the container is a polyethylene terephthalate (PET) container.

After accelerated stability testing of compositions of the invention stored in containers manufactured from high density polyethylene (HDPE) or polyethylene terephthalate (PET) and other plastic and metal components, no evidence of corrosion or other damage to the containers was noted. Thus, there is also provided a container comprising an aqueous dental appliance cleanser composition of the invention, wherein the container is manufactured from polyethylene terephthalate (PET) or high-density polyethylene (HDPE). There is also provided a dispenser comprising (i) a container comprising an aqueous dental appliance cleanser composition of the invention and (ii) a foaming applicator head, wherein the container is manufactured from polypropylene (PP) More particularly, a dispenser consisting essentially of (i) a polyethylene terephthalate (PET) or high density polyethylene (HDPE) container comprising an aqueous dental appliance cleanser composition of the invention and (ii) a foaming applicator head configured to dispense the aqueous dental appliance cleanser composition in the form of a foam. Yet more particularly, there is provided a dispenser comprising or consisting essentially of a polyethylene terephthalate (PET) or high-density polyethylene (HDPE) container defining a reservoir and a foaming applicator head in fluid communication with the reservoir, wherein the reservoir comprises a cleanser composition of the invention and wherein the foaming applicator head is configured to dispense the aqueous dental appliance cleanser composition from the reservoir in the form of a foam.

### Compositions for Use in Cleansing

The present invention is also directed toward compositions as described above for use in methods of cleansing dentures. These methods comprise applying the composition to a surface of a dental appliance and maintaining the dental appliance in contact with the composition for a period of time.

Preferably, the composition is applied in the form of a spray and/or foam. Thus, the method may comprise dispensing the composition from a container in the form of a foam. Particularly, when being dispensed, the composition is activated to a foam via a foaming applicator head. The foam is applied directly to the surface of a dental appliance, optionally with brushing. Alternatively, the composition may be applied in the form of a spray and agitated to form a foam by manual means, such as brushing.

Particularly, the composition is maintained in contact with a surface of the dental appliance for a period of time. Particularly, the composition is maintained in contact with a surface of the dental appliance for a period of time sufficient to kill at least 80%, such as at least 85%, at least 90%, at least 95%, at least 96%, at least 97%, at least 98%, at least 99% or at least 99.9% of microorganisms present on the surface of the dental appliance.

Particularly, the microorganisms present on the surface of the dental appliance include *Candida albicans* and/or *Klebsiella pneumoniae,* more particularly *Candida albicans.* Particularly, the composition is maintained in contact with a surface of the dental appliance for a period of time sufficient to achieve at least a log 2.5 reduction in the number of *Candida albicans and*/*or Klebsiella pneumoniae* colony forming units on the surface, preferably at least a log 3 reduction, more preferably at least a log 4 reduction, most preferably at least a log 5 reduction. Preferably, the period of time is no more than 3 minutes, 3 minutes or less, no more than 2 minutes, 2 minutes or less, no more than 60 seconds, 60 seconds or less, particularly from 30 seconds to 60 seconds. After this period of time, the dental appliance is rinsed with water to remove residual composition and placed into the wearer's mouth. Compositions of the invention are particularly suitable for use in cleansing dental prostheses that are cleaned and then placed into the mouth because this generally involves some manual handling of the dental appliance. Compositions of the invention have improved dermal tolerance (for example, in comparison to certain other cleansers), and are particularly suitable for cleansing dental appliances during which the cleansing composition may come into contact with the skin of the hands or fingers, either accidentally or during manual handling steps. Particularly, compositions of the invention have a dermal tolerance score of at least 10% mean cell viability, at least 11%, at least 12%, at least 13%, at least 14% or at least 15% mean cell viability as measured by in vitro dermal tolerance assay.

Cleansing methods of the invention are performed outside of the oral cavity. Thus, these methods may be referred to as methods of cleansing a dental appliance outside the oral cavity.

In certain embodiments, there is provided a composition of the invention for use in a method of cleansing dentures, the method comprising: dispensing the composition from a container in the form of a foam onto a surface of a dental appliance, maintaining the dental appliance in contact with the foam for a period of time, particularly from 30 to 60 seconds, optionally brushing and/or rinsing the dental appliance with water to remove the foam and optionally placing the dental appliance into the mouth of a subject.

In certain embodiments, there is provided a composition of the invention for use in a method of cleansing dentures, the method comprising: removing a dental appliance from the mouth of a subject, dispensing the dental appliance cleanser from a container in the form of a foam, applying the foam to a surface of the dental appliance, maintaining the dental appliance in contact with the foam for a period of time, particularly from 30 to 60 seconds, optionally brushing and/or rinsing the dental appliance with water to remove the foam and returning the dental appliance to the mouth of the subject.

### Compositions for Use in killing microorganisms

There is also provided a composition of the invention for use in a method of killing microorganisms, such as *Candida albicans,* on the surface of a dental appliance. In some embodiments, there is provided a composition of the invention, for use in a method of killing bacteria, such as *Klebsiella pneumoniae,* on the surface of a dental appliance. In some embodiments, there is provided a composition of the invention, for use in a method of killing fungus, for example yeast, such as *Candida albicans,* on the surface of a dental appliance. In some embodiments, there is provided a composition of the invention, for use in a method of inactivating, for example killing, a virus on the surface of a dental appliance.

These methods comprise applying the composition of the invention to a surface of a dental appliance, said surface comprising at least one microorganism, and maintaining the dental appliance in contact with the composition for a period of time sufficient to kill the at least one microorganism. A person skilled in the art will recognize that when the composition is applied to a surface of a dental appliance that comprises microorganisms, the composition will also contact the microorganisms on the surface of a dental appliance.

Particularly, the composition is maintained in contact with a surface of the dental appliance for a period of time sufficient to kill at least 80%, such as at least 85%, at least 90%, at least 95%, at least 96%, at least 97%, at least 98%, at least 99% or at least 99.9% of microorganisms present on the surface of the dental appliance.

Thus, the methods may comprise applying the composition of the invention to a surface of a dental appliance, said surface comprising a population of microorganisms, and maintaining the dental appliance in contact with the composition for a period of time sufficient to kill at least 80% of the population of microorganisms.

Particularly, the microorganisms are *Candida albicans* and/or *Klebsiella pneumoniae,* more particularly *Candida albicans.*

Particularly, the composition is maintained in contact with a surface of the dental appliance for a period of time sufficient to achieve at least a log 2.5 reduction in the number of *Candida albicans and*/*or Klebsiella pneumoniae* colony forming units on the surface, preferably at least a log 3 reduction, more preferably at least a log 4 reduction, most preferably at least a log 5 reduction.

With regard to viruses, particularly the compositions of the Invention are capable of decreasing the viral titer (corresponding to the content of infectious viruses present per unit volume in a cell culture lysate) of a viral strain by at least log 4. A log 4 reduction corresponds to a 10⁴-fold or 99.99% reduction of the viral strain tested. The virus may be an enveloped or non-enveloped virus. The virus may be an influenza virus, a coronavirus or a rhinovirus. In certain embodiments, the virus is respiratory syncytial virus. In certain embodiments the virus is influenza virus H1N1. In certain embodiments the virus is a coronavirus such as SARS-CoV, SARS-CoV-1 or SARS-CoV-2.

Thus, the methods may comprise applying the composition of the invention to a surface of a dental appliance, said surface comprising a population of bacteria and/or fungi, including for example *Candida albicans* and/or *Klebsiella pneumoniae,* and maintaining the dental appliance in contact with the composition for a period of time sufficient to achieve a reduction in the number of colony forming units of bacteria and/or fungi, for example *Candida albicans* and/or *Klebsiella pneumoniae,* of at least 80% or equivalent to at least log 2.5.

Preferably, the period of time is from about 30 seconds to no more than 3 minutes, 3 minutes or less, no more than 2 minutes, 2 minutes or less, no more than 60 seconds, 60 seconds or less, particularly from about 30 seconds to about 3 minutes, from about 30 seconds to about 2 minutes, preferably from about 30 seconds to about 60 seconds. After this period of time, the dental appliance is rinsed with water to remove residual composition and placed into the wearer's mouth.

Particularly, compositions of the invention have a dermal tolerance score of at least 10% mean cell viability, at least 11%, at least 12%, at least 13%, at least 14% or at least 15% mean cell viability as measured by in vitro dermal tolerance assay.

In certain embodiments, there is provided a composition of the invention for use in a method of killing microorganisms, such as *Candida albicans,* on the surface of a dental appliance, the method comprising: dispensing the composition from a container in the form of a foam, applying the foam to a surface of a dental appliance, said surface comprising microorganisms, maintaining the dental appliance in contact with the foam for a period of time sufficient to kill the microorganisms, particularly from 30 to 60 seconds, optionally brushing and/or rinsing the dental appliance with water to remove the foam and optionally placing the dental appliance into the mouth of a subject.

Particularly, the surface of the dental appliance comprises a population of microorganisms, such as *Candida albicans.* More particularly, the dental appliance is maintained in contact with the foam for a period of time sufficient to kill period at least 80%, such as at least 85%, at least 90%, at least 95%, at least 96%, at least 97%, at least 98%, at least 99% or at least 99.9% of microorganisms present on the surface of the dental appliance. Yet more particularly, at least 80%, such as at least 85%, at least 90%, at least 95%, at least 96%, at least 97%, at least 98%, at least 99% or at least 99.9% of microorganisms present on the surface of the dental appliance are killed within 30 to 60 seconds. Still yet more particularly, at least 80%, such as at least 85%, at least 90%, at least 95%, at least 96%, at least 97%, at least 98%, at least 99% or at least 99.9% of *Candida albicans* CFUs present on the surface of the dental appliance are killed within 30 to 60 seconds.

In certain embodiments, there is provided a composition of the invention for use in a method of killing microorganisms, such as *Candida albicans,* on the surface of a dental appliance, the method comprising: removing a dental appliance from the mouth of a subject, dispensing the composition from a container in the form of a foam onto a surface of the dental appliance, said surface comprising microorganisms, maintaining the dental appliance in contact with the foam for a period of time sufficient to kill the microorganisms, particularly from 30 to 60 seconds, rinsing the dental appliance with water, optionally with brushing, to remove the foam and returning the dental appliance to the mouth of the subject.

### Use of Compositions in methods of cleansing

The present invention further includes methods of cleansing a dental appliance, such as a partial or full denture, using a composition as described above.

For example, there is provided a method of cleansing dentures, the method comprising: dispensing a composition of the invention from a container in the form of a spray and/or foam onto a surface of a dental appliance, maintaining the dental appliance in contact with the foam for a period of time, particularly from 30 to 60 seconds, rinsing the dental appliance with water, optionally with brushing, to remove residual composition and optionally placing the dental appliance into the mouth of a subject.

In some embodiments, there is provided a method of cleansing dentures, the method comprising: removing a dental appliance from the mouth of a subject, dispensing a composition of the invention from a container in the form of a foam onto a surface of the dental appliance, maintaining the dental appliance in contact with the foam for a period of time, particularly from 30 to 60 seconds, rinsing the dental appliance with water, optionally with brushing, to remove residual composition and returning the dental appliance to the mouth of the subject.

### Use of Compositions in methods of killing microorganisms

There is also provided a method of killing microorganisms, such as *Candida albicans,* on the surface of a dental appliance using a composition of the invention.

There is further provided a method of killing microorganisms, such as *Candida albicans,* on the surface of a dental appliance, the method comprising: dispensing a composition according to the invention from a container in the form of a foam onto a surface of a dental appliance, said surface comprising microorganisms, maintaining the dental appliance in contact with the foam for a period of time sufficient to kill the microorganisms, particularly from 30 to 60 seconds, rinsing the dental appliance with water, optionally with brushing, to remove the foam and optionally placing the dental appliance into the mouth of a subject.

There is further provided a method of killing microorganisms, such as *Candida albicans,* on the surface of a dental appliance, the method comprising: removing a dental appliance from the mouth of a subject, dispensing a composition according to the invention from a container in the form of a foam, applying the foam to a surface of the dental appliance, said surface comprising microorganisms, maintaining the dental appliance in contact with the foam for a period of time sufficient to kill the bacteria, particularly from 30 to 60 seconds, rinsing the dental appliance with water, optionally with brushing, to remove the foam and returning the dental appliance to the mouth of the subject.

### General

The term "comprising" encompasses "including" e.g. a composition "comprising" X may include something additional e.g. X + Y. In some implementations, the term "comprising" refers to the inclusion of the indicated active agent, such as recited compounds, as well as inclusion of other active agents, and carriers, excipients, emollients, stabilizers, etc., known in the consumer health industry and generally recognized as safe (GRAS). Use of the transitional phrase "consisting essentially of" means that the scope of a claim is to be interpreted to encompass the specified materials or steps recited in the claim, and those that do not materially affect the basic and novel characteristic(s) of the claimed invention. See, In re Herz, 537 F.2d 549, 551-52, 190 USPQ 461, 463 (CCPA 1976) (emphasis in the original); see also MPEP § 2111.03. Thus, the term "consisting essentially of" when used in a claim of this invention is not intended to be interpreted to be equivalent to "comprising". The term "consisting of" and variations thereof means including and limited to (for example, the specific recited constituents or steps). In certain territories, the term "comprising an active ingredient consisting of" may be used in place of "consisting essentially". The term "about" in relation to a numerical value x is optional and means, for example, that the numerical value may comprise some variance around the stated number to allow for routine experimental fluctuation, measurement variance or to encompass minor deviations that may achieve substantially the same results as the stated number, such as x±10%, x±5%, x±4%, x±3%, x±2% or x±1%. The word "substantially" does not exclude "completely" e.g. a composition which is "substantially free" from Y may be completely free from Y. Where necessary, the word "substantially" may be omitted from the definition of the invention. All percentages and ratios used herein are by weight of total composition, unless otherwise indicated.

All references or patent applications cited within this patent specification are incorporated by reference herein.

### Embodiments of the Invention

The following clauses describe certain embodiments of the invention:
E1) An aqueous storage-stable dental appliance cleanser composition comprising: from about 0.05% to about 4% by weight of hydrogen peroxide, from about 0.01% to about 0.25% by weight of at least one acid selected from the group consisting of salicylic acid, furoic acid and formic acid, from about 0.05% to about 2.00% by weight of an anionic surfactant.
E2) An aqueous storage-stable dental appliance cleanser composition comprising or consisting essentially of: from about 0.05% to about 4% by weight of hydrogen peroxide, from about 0.01% to about 0.25% by weight of at least one acid selected from the group consisting of salicylic acid, furoic acid and formic acid, from about 0.05% to about 2.00% by weight of an anionic surfactant wherein the composition has a pH within the range of from 2.8 to 3.5.
E3) An aqueous storage-stable dental appliance cleanser composition comprising or consisting essentially of: from about 0.05% to about 4% by weight of hydrogen peroxide, from about 0.01% to about 0.25% by weight of at least one acid selected from the group consisting of salicylic acid, furoic acid and formic acid, from about 0.05% to about 2.00% by weight of an anionic surfactant, from about 0.01% to about 0.08% by weight of a buffer particularly, a sodium citrate buffer, wherein the composition has a pH within the range of from 2.8 to 3.5.
E4) An aqueous storage-stable dental appliance cleanser composition comprising or consisting essentially of: from about 0.05% to about 4% by weight of hydrogen peroxide, from about 0.01% to about 0.25% by weight of at least one acid selected from the group consisting of salicylic acid, furoic acid and formic acid, from about 0.05% to about 2.00% by weight of an anionic surfactant, from about 0.01% to about 0.08% by weight of a buffer particularly, a sodium citrate buffer, wherein the composition has a pH within the range of from 2.8 to 3.5 and wherein the composition further comprises from about 0.01% to about 0.5% by weight of a sensory component.
E5) An aqueous storage-stable dental appliance cleanser composition comprising or consisting essentially of: from about 0.05% to about 4% by weight of hydrogen peroxide, from about 0.01% to about 0.25% by weight of 2- furoic acid and/or 3-furoic acid, from about 0.05% to about 2.00% by weight of an anionic surfactant wherein the composition has a pH within the range of from 2.8 to 3.5.
E6) An aqueous storage-stable dental appliance cleanser composition comprising or consisting essentially of: from about 0.05% to about 4% by weight of hydrogen peroxide, from about 0.01% to about 0.25% by weight of formic acid, from about 0.05% to about 2.00% by weight of an anionic surfactant wherein the composition has a pH within the range of from 2.8 to 3.5.
E7) An aqueous storage-stable dental appliance cleanser composition comprising or consisting essentially of: from about 0.05% to about 4% by weight of hydrogen peroxide, from about 0.01% to about 0.25% by weight of salicylic acid, from about 0.05% to about 2.00% by weight of an anionic surfactant wherein the composition has a pH within the range of from 2.8 to 3.5.
E8) An aqueous storage-stable dental appliance cleanser composition comprising or consisting essentially of: from about 0.1% to about 2% by weight of hydrogen peroxide, from about 0.01% to about 0.25% by weight of salicylic acid, from about 0.05% to about 2.00% by weight of an anionic surfactant wherein the composition has a pH within the range of from 2.8 to 3.5.
E9) An aqueous storage-stable dental appliance cleanser composition comprising or consisting essentially of: from about 0.1% to about 2% by weight of hydrogen peroxide, from about 0.01% to about 0.25% by weight of salicylic acid, from about 0.25% to about 1.00% by weight of an anionic surfactant wherein the composition has a pH within the range of from 2.8 to 3.5.
E10) An aqueous storage-stable dental appliance cleanser composition comprising or consisting essentially of: from about 0.1% to about 2% by weight of hydrogen peroxide, from about 0. 1% to about 0.2% by weight of salicylic acid, from about 0.25% to about 1.00% by weight of an anionic surfactant wherein the composition has a pH within the range of from 2.8 to 3.5.
E11) An aqueous storage-stable dental appliance cleanser composition comprising or consisting essentially of: from about 0.25% to about 1% by weight of hydrogen peroxide, from about 0.01% to about 0.25% by weight of salicylic acid, from about 0.05% to about 2.00% by weight of an anionic surfactant wherein the composition has a pH within the range of from 2.8 to 3.5.
E12) An aqueous storage-stable dental appliance cleanser composition comprising or consisting essentially of: from about 0.25% to about 1% by weight of hydrogen peroxide, from about 0.1% to about 0.2% by weight of salicylic acid, from about 0.25% to about 1.00% by weight of an anionic surfactant wherein the composition has a pH within the range of from 2.8 to 3.5.
E13) An aqueous storage-stable dental appliance cleanser composition comprising or consisting essentially of: from about 0.05% to about 4% by weight of hydrogen peroxide, from about 0.01% to about 0.25% by weight of salicylic acid, from about 0.05% to about 2.00% by weight of an anionic surfactant, from about 0.01% to about 0.08% by weight of a buffer particularly, a sodium citrate buffer, wherein the composition has a pH within the range of from 2.8 to 3.5.
E14) An aqueous storage-stable dental appliance cleanser composition comprising or consisting essentially of: from about 0.1% to about 2% by weight of hydrogen peroxide, from about 0. 1% to about 0.2% by weight of salicylic acid, from about 0.25% to about 1.00% by weight of an anionic surfactant, from about 0.01% to about 0.08% by weight of a buffer particularly, a sodium citrate buffer wherein the composition has a pH within the range of from 2.8 to 3.5.
E15) An aqueous storage-stable dental appliance cleanser composition comprising or consisting essentially of: from about 0.1% to about 2% by weight of hydrogen peroxide, from about 0. 1% to about 0.2% by weight of salicylic acid, from about 0.25% to about 1.00% by weight of an anionic surfactant, from about 0.02% to about 0.06% by weight of a buffer particularly, a sodium citrate buffer wherein the composition has a pH within the range of from 2.8 to 3.5.
E16) An aqueous storage-stable dental appliance cleanser composition comprising or consisting essentially of: from about 0.1% to about 2% by weight of hydrogen peroxide, from about 0. 1% to about 0.2% by weight of salicylic acid, from about 0.25% to about 1.00% by weight of an anionic surfactant, from about 0.02% to about 0.08% by weight of a sodium citrate buffer wherein the composition has a pH within the range of from 2.8 to 3.5.
E17) An aqueous storage-stable dental appliance cleanser composition comprising or consisting essentially of: from about 0.25% to about 1% by weight of hydrogen peroxide, from about 0.1% to about 0.2% by weight of salicylic acid, from about 0.25% to about 1.00% by weight of an anionic surfactant, from about 0.01% to about 0.08% by weight of a buffer particularly, a sodium citrate buffer wherein the composition has a pH within the range of from 2.8 to 3.5.
E18) An aqueous storage-stable dental appliance cleanser composition comprising or consisting essentially of: from about 0.25% to about 1% by weight of hydrogen peroxide, from about 0.1% to about 0.2% by weight of salicylic acid, from about 0.25% to about 1.00% by weight of an anionic surfactant, from about 0.02% to about 0.08% by weight of a buffer particularly, a sodium citrate buffer wherein the composition has a pH within the range of from 2.8 to 3.5.
E19) An aqueous storage-stable dental appliance cleanser composition comprising or consisting essentially of: from about 0.25% to about 1% by weight of hydrogen peroxide, from about 0.1% to about 0.2% by weight of salicylic acid, from about 0.25% to about 1.00% by weight of an anionic surfactant, from about 0.02% to about 0.08% by weight of a sodium citrate buffer wherein the composition has a pH within the range of from 2.8 to 3.5.
E20) An aqueous storage-stable dental appliance cleanser composition comprising or consisting essentially of: from about 0.05% to about 4% by weight of hydrogen peroxide, from about 0.01% to about 0.25% by weight of salicylic acid, from about 0.05% to about 2.00% by weight of an anionic surfactant, from about 0.01% to about 0.08% by weight of a buffer particularly, a sodium citrate buffer, wherein the composition has a pH within the range of from 2.8 to 3.5 and wherein the composition further comprises from about 0.01% to about 0.5% by weight of a sensory component.
E21) An aqueous storage-stable dental appliance cleanser composition comprising or consisting essentially of: from about 0.1% to about 2% by weight of hydrogen peroxide, from about 0.1% to about 0.2% by weight of salicylic acid, from about 0.25% to about 1.00% by weight of an anionic surfactant, from about 0.02% to about 0.08% by weight of a sodium citrate buffer wherein the composition has a pH within the range of from 2.8 to 3.5 and wherein the composition further comprises from about 0.01% to about 0.5% by weight of a sensory component.
E22) An aqueous storage-stable dental appliance cleanser composition comprising or consisting essentially of: from about 0.1% to about 2% by weight of hydrogen peroxide, from about 0. 1% to about 0.2% by weight of salicylic acid, from about 0.25% to about 1.00% by weight of an anionic surfactant, from about 0.02% to about 0.08% by weight of a sodium citrate buffer wherein the composition has a pH within the range of from 2.8 to 3.5 and wherein the composition further comprises from about 0.1% to about 0.3% by weight of a sensory component.
E23) An aqueous storage-stable dental appliance cleanser composition comprising or consisting essentially of: from about 0.25% to about 1% by weight of hydrogen peroxide, from about 0.1% to about 0.2% by weight of salicylic acid, from about 0.25% to about 1.00% by weight of an anionic surfactant, from about 0.02% to about 0.08% by weight of a sodium citrate buffer wherein the composition has a pH within the range of from 2.8 to 3.5 and wherein the composition further comprises from about 0.01% to about 0.5% by weight of a sensory component.
E24) An aqueous storage-stable dental appliance cleanser composition comprising or consisting essentially of: from about 0.25% to about 1% by weight of hydrogen peroxide, from about 0. 1% to about 0.2% by weight of salicylic acid, from about 0.25% to about 1.00% by weight of an anionic surfactant, from about 0.02% to about 0.08% by weight of a sodium citrate buffer wherein the composition has a pH within the range of from 2.8 to 3.5 and wherein the composition further comprises from about 0.1% to about 0.3% by weight of a sensory component.
E25) An aqueous storage-stable dental appliance cleanser composition comprising or consisting essentially of: from about 0.25% to about 1% by weight of hydrogen peroxide, from about 0. 1% to about 0.2% by weight of salicylic acid, from about 0.25% to about 1.00% by weight of sodium lauryl sulfate, from about 0.02% to about 0.08% by weight of a sodium citrate buffer wherein the composition has a pH within the range of from 2.8 to 3.5 and wherein the composition further comprises from about 0.1% to about 0.3% by weight of a sensory component.
E26) An aqueous storage-stable dental appliance cleanser composition comprising or consisting essentially of: from about 0.25% to about 1% by weight of hydrogen peroxide, from about 0.01% to about 0.25% by weight of 2-furoic acid and/or 3-furoic acid, from about 0.25% to about 1.00% by weight of an anionic surfactant, from about 0.02% to about 0.08% by weight of a sodium citrate buffer wherein the composition has a pH within the range of from 2.8 to 3.5 and wherein the composition further comprises from about 0.1% to about 0.3% by weight of a sensory component.
E27) An aqueous storage-stable dental appliance cleanser composition comprising or consisting essentially of: from about 0.25% to about 1% by weight of hydrogen peroxide, from about 0.01% to about 0.25% by weight of 2-furoic acid and/or 3-furoic acid, from about 0.25% to about 1.00% by weight of sodium lauryl sulfate, from about 0.02% to about 0.08% by weight of a sodium citrate buffer wherein the composition has a pH within the range of from 2.8 to 3.5 and wherein the composition further comprises from about 0.1% to about 0.3% by weight of a sensory component.
E28) An aqueous storage-stable dental appliance cleanser composition comprising or consisting essentially of: from about 0.25% to about 1% by weight of hydrogen peroxide, from about 0.01% to about 0.25% by weight of formic acid, from about 0.25% to about 1.00% by weight of an anionic surfactant, from about 0.02% to about 0.08% by weight of a sodium citrate buffer wherein the composition has a pH within the range of from 2.8 to 3.5 and wherein the composition further comprises from about 0.1% to about 0.3% by weight of a sensory component.
E29) An aqueous storage-stable dental appliance cleanser composition comprising or consisting essentially of: from about 0.25% to about 1% by weight of hydrogen peroxide, from about 0.01% to about 0.25% by weight of formic acid, from about 0.25% to about 1.00% by weight of sodium lauryl sulfate, from about 0.02% to about 0.08% by weight of a sodium citrate buffer wherein the composition has a pH within the range of from 2.8 to 3.5 and wherein the composition further comprises from about 0.1% to about 0.3% by weight of a sensory component.
E30) A dispenser comprising or consisting essentially of, a container defining a reservoir and a dispenser head in fluid communication with the reservoir, wherein the reservoir comprises a composition according to any one of Embodiments E1 to E29, and wherein the dispenser head is configured to dispense the composition from the reservoir in the form of a spray and/or foam.
E31) The dispenser of Embodiment E30, wherein the dispenser head is a foaming applicator head configured to dispense the composition in the form of a foam.

In order that this invention may be better understood, the following examples are set forth. These examples are for purposes of illustration only and are not to be construed as limiting the scope of the invention in any manner.

### EXAMPLES

### Example 1

Aqueous compositions containing 0.5% hydrogen peroxide, 0.5% sodium lauryl sulfate, an acid (see Table 1 for amounts) and water (balance to 100%) were prepared and tested for in vitro anti-microbial efficacy against *Candida albicans,* an opportunistic pathogenic yeast.

Testing was conducted by adding the compositions to a planktonic suspension of *Candida albicans* (1-5 ×10⁸ cfu/ml) and then neutralising after 60 seconds contact time. The log reduction in *Candida albicans* was then calculated for each composition, with a performance target of > log 5 reduction (kills 99.999%).

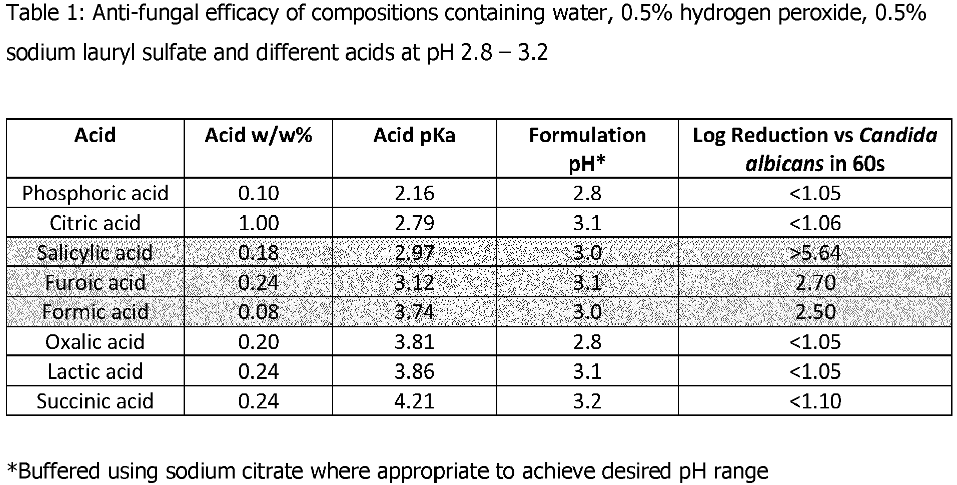

Of these compositions, only those comprising salicylic acid, furoic acid or formic acid were capable of achieving a log 2.50 or greater reduction in colony forming units of C. *albicans.* Surprisingly, compositions comprising 0.18% of salicylic acid yielded a > log 5 reduction (equivalent to 99.99% killing). Other acids tested (phosphoric, citric, oxalic, lactic and succinic acids) achieved <1.10 log reduction demonstrating low levels of anti-microbial efficacy. There was no correlation between acid pKa and anti-bacterial efficacy.

### Example 2

The composition containing salicylic acid was then tested against *Klebsiella pneumoniae,* a gram-negative bacteria that causes multiple healthcare-associated infections.

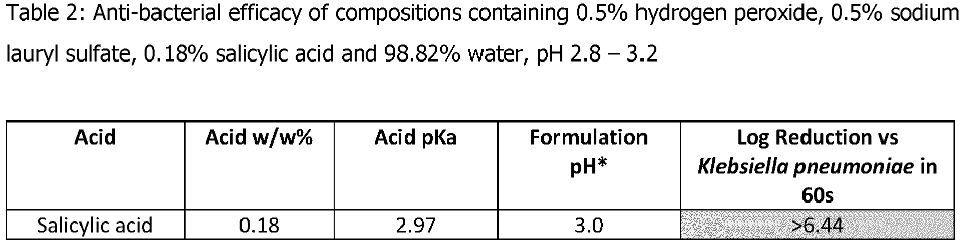

These results confirmed the surprisingly strong anti-bacterial efficacy of compositions comprising salicylic acid.

### Example 3

Compositions containing water, 0.5% hydrogen peroxide, 0.5% sodium lauryl sulfate and salicylic acid were prepared at different pH's and placed into storage at 40°C for 3 months to determine a suitable pH range for formulation stability.

Aged compositions were tested for in vitro antimicrobial efficacy against *Candida albicans* (60s contact time as above), as well as foam appearance after dispensing the liquid composition through a foaming-pump packaging.

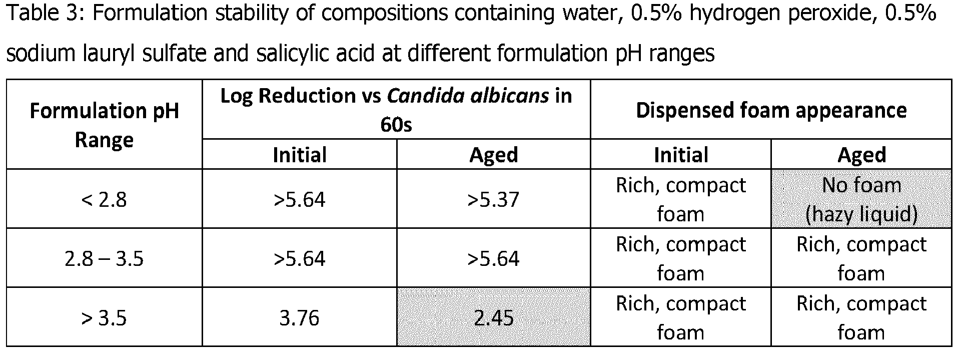

It was observed that a formulation pH of below pH 2.8 impacted foaming, resulting in a loss of foamability after sample aging. Without wishing to be bound by theory, it is believed that the sodium lauryl sulfate is degraded into insoluble lauryl alcohol below pH 2.8, resulting in a gradual loss of foamability over time.

It was also observed that formulations having a pH above 3.5 had a lower initial antimicrobial efficacy (3.76 log Reduction vs *Candida albicans* in 60s) which declined during storage. Again, without wishing to be bound by theory, it is believed that the hydrogen peroxide begins to degrade at less acidic pH's, resulting in a gradual loss of efficacy over time.

Accordingly, it was determined that the optimum pH for stability and antimicrobial efficacy was within the range from pH 2.8 to pH 3.5.

### Example 4

A composition containing 0.5% hydrogen peroxide, 0.5% sodium lauryl sulfate, 0.18% salicylic acid and 98.82% water (pH 2.8 - 3.2) was prepared and compared with an existing dental appliance cleanser product (FRESHDENT denture foam, EverSmile). FRESHDENT contains the following constituents: Water, 3.75% hydrogen peroxide, glycerine, PVP, PEG, sodium lauryl sulfate, sucralose, sodium citrate, sodium benzoate, etidronic acid, mentha arvensis (mint) oil.

In vitro dermal tolerance was measured using the commercial in vitro EPIDERM skin irritation test (EPI-200-SIT, MatTek Corporation), also described in Kandarova et al (Pre-validation of an in vitro skin irritation test for medical devices using the reconstructed human tissue model EpiDerm™ Toxicol. In Vitro. 2018;50:407-417). In brief, a 3D tissue model of normal, human-derived epidermal keratinocytes (NHEK) were cultured and cell viability measured using a well-known cell viability assay. Cultures were then topically exposed to the cleanser solutions for a period of 60 seconds and cell viability re-measured to determine mean % cell viability post product exposure. A target remaining cell viability was set at 2.71%, as this represents the in vitro performance of a control GSK product that has been in the market for over 10 years with an excellent actual dermal tolerance profile, according to GSK Adverse Events tracking. Accordingly, any viability above 2.71% is expected to have a similar or better in-market dermal tolerance profile.

Material compatibility studies were conducted on a broad range of denture materials, including polymethyl methacrylate, polyamide, stainless steel, silver solder, ethylenevinyl acetate copolymer, thermoplastic polyurethane, cobalt-chromium alloy and polypropylene. Materials were repeatedly exposed to compositions for 60 seconds with brushing and then rinsing, over multiple cycles to represent 2 years simulated daily usage. Materials were then assessed using light microscopy and electron microscopy for surface damage. Material integrity post-exposure was visually graded as either structurally damaged (poor compatibility), minor superficially damaged (moderate/good compatibility) or not damaged (excellent compatibility).

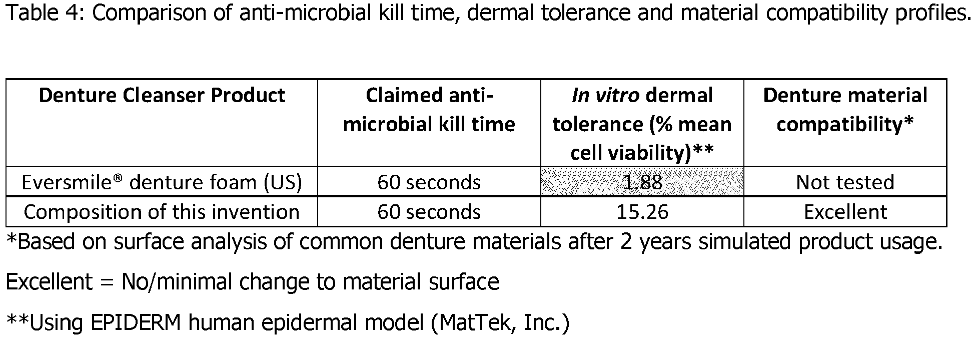

Surprisingly, the composition of the invention provided fast antimicrobial efficacy within 60 seconds (convenience) despite having a low-level of hydrogen peroxide compared to the FRESHDENT product (0.5% vs 3.75%). The composition of the invention also provided improved dermal tolerance (safety) compared to the FRESHDENT product whilst also having excellent denture material compatibility.

### Example 5

The stain removal properties of the composition were evaluated against a commercially available toothpaste, often used to clean dental appliances, in a simulated stain removal test. Ceramic tiles were stained with a concentrated tea solution. Half of the tile was treated with either (a) the composition or (b) toothpaste and the stained area was brushed, rinsed and dried (Figure 1). Compositions of the invention were better at removing stains than the commercial toothpaste.

In view of this guidance, one of ordinary skill in the art will be able to adjust the parameters provided in the Examples to achieve similar results within the remit of the claims.

## Claims

1. An aqueous storage-stable dental appliance cleanser composition comprising: (a) from about 0.05% to about 4% by weight of hydrogen peroxide, (b) from about 0.01% to about 0.25% by weight of at least one acid selected from the group consisting of salicylic acid, fuoric acid and formic acid, (c) from about 0.05% to about 2.00% by weight of an anionic surfactant.

2. The dental appliance cleanser composition of Claim 1, wherein the composition has a pH of from 2.8 to 3.5.

3. The dental appliance cleanser composition of Claim 1 or Claim 2, wherein the composition further comprises (d) a means for adjusting or maintaining the pH of the composition.

4. The dental appliance cleanser composition of Claim 3, wherein the means for adjusting or maintaining the pH of the composition is a pH buffer.

5. The dental appliance cleanser composition of Claim 4 which comprises from about 0.01% to about 0.08% by weight of the pH buffer.

6. The dental appliance cleanser composition of Claim 4 or 5, wherein the pH buffer is sodium citrate buffer.

7. The dental appliance cleanser composition of any one of Claims 1 to 6, wherein the acid is salicylic acid.

8. The dental appliance cleanser composition of any one of Claims 1 to 7, which comprises from about 0.1% to about 2% by weight of hydrogen peroxide, particularly from about 0.25% to 1% by weight of hydrogen peroxide.

9. The dental appliance cleanser composition of Claim 7 or 8, wherein the anionic surfactant is a sodium alkyl sulfate.

10. The dental appliance cleanser composition of Claim 9, wherein the sodium alkyl sulfate is selected from the group consisting of sodium lauryl sulfate, sodium lauryl sulfonate and sodium laureth sulfate.

11. The dental appliance cleanser composition of Claim 1 which consists essentially of: (a) from about 0.1% to about 2% by weight of hydrogen peroxide, (b) from about 0.01% to about 0.25% by weight of salicylic acid, (c) from about 0.05% to about 2.00% by weight of an anionic surfactant selected from the group consisting of sodium lauryl sulfate, sodium lauryl sulfonate and sodium laureth sulfate, and (d) a pH buffer, wherein the composition has a pH of from 2.8 to 3.5 and wherein the composition comprises at least 90%, preferably 95%, by weight of water.

12. The dental appliance cleanser composition of any preceding claim, further comprising (e) a sensory component, particularly a flavouring and/or colouring agent.

13. The dental appliance cleanser composition according to any preceding claim, for use in a method of cleansing dentures.

14. The dental appliance cleanser composition according to any one of claims 1 to 12, for use in a method of killing microorganisms such as *Candida albicans* on the surface of a dental appliance comprising, contacting the microorganisms on the surface of the dental appliance with the composition for a period of time sufficient to kill the microorganisms.

15. A dispenser comprising or consisting essentially of (i) a container defining a reservoir and (ii) a dispenser head in fluid communication with the reservoir, wherein the reservoir comprises a dental appliance cleanser composition according to any one of claims 1 to 12 and wherein the dispenser head is configured to dispense the aqueous dental appliance cleanser composition from the reservoir in the form of a spray and/or foam.
